(19) Europäisches Patentamt
European Patent Office
Office européen des brevets

(11) EP 4 650 754 A1

(12) **EUROPEAN PATENT APPLICATION**

(43) Date of publication:
**19.11.2025 Bulletin 2025/47**

(21) Application number: **24175513.1**

(22) Date of filing: **13.05.2024**

(51) International Patent Classification (IPC):
**G01N 21/3563** (2014.01)    **G01N 21/3577** (2014.01)
**G01N 21/359** (2014.01)    **G01N 21/84** (2006.01)
**G01N 21/35** (2014.01)

(52) Cooperative Patent Classification (CPC):
**G01N 21/359; G01N 21/3563; G01N 21/3577;**
**G01N 33/442;** G01N 2021/3595; G01N 2021/8416;
G01N 2201/1293

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Borealis GmbH**
**1020 Vienna (AT)**

(72) Inventors:
• **Goswami, Mithun**
 **4021 Linz (AT)**
• **Hettrich-Keller, Michael**
 **4021 Linz (AT)**

(74) Representative: **Ter Meer Steinmeister & Partner**
**Patentanwälte mbB**
**Nymphenburger Straße 4**
**80335 München (DE)**

(54) **METHOD FOR CALIBRATION, METHOD FOR IN-LINE QUALITY CONTROL IN A COMPOUNDING PROCESS BASED ON NIR AND APPARATUS WITH NIR QUALITY CONTROL**

(57) A method for calibration using in-line FT-NIR spectral information and known values of at least one analytical feature, such as a content of a polymeric contaminant or a non-polymeric contaminant, of a polymer or polymer composition is disclosed. Furthermore, a method for in-line quality control in a compounding process based on FT-NIR spectral information is provided. Additionally, an apparatus for extrusion and pelletizing under in-line FT-NIR quality control is described. The apparatus 100 comprises an extruder 10 having a feed zone 11, a melting zone 13, a kneading zone 14, and a discharge zone 15, a pelletizer 20 having a cutter 21 for cutting the extruded melt 61 into pellets 62 and a collection station 22 for collecting the pellets, and an in-line FT-NIR sensor 30 installed outside of the extruder and outside of the pelletizer to obtain spectral information of the polymer or polymer composition from the extruded melt or the pellets. The apparatus may further comprise a cooling bath 40 between the extruder and the pelletizer.

**Figure 9**

EP 4 650 754 A1

**Description**

**Field of the Invention**

The present invention relates to a method for calibration using in-line FT-NIR spectral information and known values of at least one analytical feature of a polymer or polymer composition. Furthermore, a method for in-line quality control in a compounding process based on FT-NIR spectral information is provided. Additionally, the present invention is directed to an apparatus for extrusion and pelletizing under in-line FT-NIR quality control.

**Technical Background**

Polymer compounding is the process of blending different polymers or mixing polymers with non-polymeric additives. Conventionally, the quality of the resulting polymers or polymer compositions is determined after the completion of compounding. However, this off-line quality control is time consuming and does not allow for real-time monitoring and adjustment of the compounding process parameters. Moreover, off-line quality control does not enable the operator to quickly differentiate between high- and low-quality compounds. Hence, it is desirable to develop a method for in-line quality monitoring which can help to meet these requirements.

Near-infrared (NIR) spectroscopy is a spectroscopic method that uses the near-infrared region of the electromagnetic spectrum (from about 800 nm to 2500 nm). Instrumentation for NIR spectroscopy usually comprises an infrared light source, a detector, and a dispersive element (e.g., a prism, a diffraction grating) to allow the intensity at different wavelengths to be recorded. In cases where acquisition of spectral data needs to be fast, Fourier transform NIR (FT-NIR) instruments using an interferometer are used to collect the response for all wavelengths simultaneously. Depending on the sample, the spectrum can be measured in either reflection or transmission. Current applications of NIR spectroscopy include medical, pharmaceutical, and food and agrochemical quality control.

WO2015195479 A1, for example, discloses using in-line NIR spectroscopy to analyze comminuted products such as grain particulates flowing in a moving stream. A sample of the particulate material is passed through the detection region of the NIR sensor, where the detection region is placed downstream from a unit operation that modifies the particles in the flowing stream.

ES2346494 A1 describes an in-line IR or NIR sensor in a system for determining the fat content of milk. The system is suitable for use in milk production facilities.

CN201067517 Y discloses a dropping pill machine where an NIR sensor is arranged on the material device, the trickle device, or the condensate. The NIR signal is used to determine the uniform state of the fluid, the stability of the fluid nature, the effectiveness of the condensate liquid to condensation forming, the pill shape and the contents of effective ingredients after the dropping pills form.

Witschnigg, Andreas, et al. "In-line characterization of polypropylene nanocomposites using FT-NIR." Journal of applied polymer science 117.5 (2010): 3047-3053 describes an in-line NIR spectroscopic method to characterize polypropylene nanocomposites. The dispersion of the organo-clay fillers in the polypropylene matrix is monitored directly in the melt by installing the FT-NIR probe in the extruder right before the die.

Barbas, J. M., A. V. Machado, and J. A. Covas. "In-line near-infrared spectroscopy for the characterization of dispersion in polymer-clay nanocomposites." Polymer testing 31.4 (2012): 527-536 also describes in-line NIR spectroscopy techniques to monitor the dispersion of an organo-clay filler in a polypropylene matrix. The installation of the NIR probe in the front plate of the mixing chamber allows the dispersion to be monitored during melt-mixing.

Saerens, Lien, et al. "In-line NIR spectroscopy for the understanding of polymer-drug interaction during pharmaceutical hot-melt extrusion." European Journal of Pharmaceutics and Biopharmaceutics 81.1 (2012): 230-237 describes the application of NIR spectroscopy for the in-line determination of drug concentration, polymer-drug solid-state behaviour and molecular interactions during hot-melt extrusion. The FT-NIR probe is mounted in the extrusion die.

Fischer, D., et al. "In-line process monitoring on polymer melts by NIR-spectroscopy." Fresenius' journal of analytical chemistry 359 (1997): 74-77 describes the use of in-line NIR to quantify polypropylene and ethylene vinyl acetate copolymer blends. The quantification is carried out in real-time by directly adapting the NIR probe to the extruder head.

Fischer, Dieter, et al. "Process monitoring of polymers by in-line ATR-IR, NIR and Raman spectroscopy and ultrasonic measurements." Comptes Rendus Chimie 9.11-12 (2006): 1419-1424 describes using NIR spectroscopy for real-time monitoring of the composition of a polyethylene/polystyrene blend during extrusion by placing the NIR probe in the melt-at-die interface.

NIR spectroscopy is highly dependent on process parameters such as temperature. The temperature and other fluctuating conditions of an extrusion process are therefore assumed to have a considerable effect on the NIR calibration and monitoring of the polymer compounding as described in the prior art. Therefore, there is a need for real-time in-line monitoring of polymers or polymer compositions that is not dependent on the melt temperature or other parameters of the compounding process. Furthermore, it is generally desirable to offer in-line quality control which is reliable and completely non-invasive.

**Summary of the Invention**

The above identified needs are satisfied by the method for calibration according to independent claim 1, the method for in-line quality control according to independent claim 8, and the apparatus for extrusion and pelletizing according to independent claim 12. Advantageous embodiments may be derived from the dependent claims.

**Detailed Description of the Embodiments**

The method for calibration according to the present invention comprises the following steps: (a) preparing a melt of a polymer or polymer composition of at least one polymer and optional additives in an extruder, (b) extruding the melt through a die of the extruder, (c) cutting the extruded melt into pellets, and (d) collecting the pellets, wherein the polymer or polymer composition has a known value of at least one analytical feature, the method being characterized in that it further comprises (e) obtaining spectral information of the polymer or polymer composition from the extruded melt or the pellets in steps (b) to (d) with an FT-NIR sensor, (f) repeating steps (a) to (e) for at least one further polymer or polymer composition having a known value of the at least one analytical feature, (g) using at least a part of the spectral information of the polymers or polymer compositions for establishing a calibration curve.

In other words, the method for calibration according to the present invention comprises a polymer compounding process having the following steps (a) to (d): (a) preparing a melt of a first polymer or polymer composition of at least one polymer and optional additives in an extruder, (b) extruding the melt through a die of the extruder, (c) cutting the extruded melt into pellets, and (d) collecting the pellets, wherein the polymer or polymer composition has a known value of at least one analytical feature, and the method further comprises the following steps (e) to (g): (e) obtaining spectral information of the polymer or polymer composition from the extruded melt or the pellets in steps (b) to (d) with an FT-NIR sensor, (f) repeating steps (a) to (e) for at least one further polymer or polymer composition having a known value of the at least one analytical feature, (g) using at least a part of the spectral information of the polymers or polymer compositions for establishing a calibration curve. The first polymer or polymer composition and the at least one further polymer or polymer composition may together be referred to as a first set of different polymers or polymer compositions with known values of at least one analytical feature. Likewise, the first polymer or polymer composition and the at least one further polymer or polymer composition may together be referred to as training samples or calibration samples.

In a preferred embodiment, the spectral information of the polymer or polymer composition is obtained in step (c) or step (d). In these steps, the extruded melt has already solidified and the spectral information is less affected by temperature variations. Even more preferably, the spectral information of the polymer or polymer composition is obtained in step (d). In the latter setup, the spectral information may be said to be even more meaningful since temperature variations are less likely to occur with the pellets being already much closer to ambient temperature than the extruded melt. Hence, based on the spectral information collected in one of steps (c) and (d), particular in step (d), more accurate calibration curves may be established.

Polymer compounding process

The polymer compounding process comprises steps (a) through (d). Viewed differently, steps (a) through (d) in the method for calibration according to the present invention may be said to belong to a polymer compounding process.

Polymer compounding processes typically allow the preparation of polymer formulations. Polymer formulations may be obtained by blending or melt-mixing polymeric starting materials, such as different polymers, different polymer compositions or a polymer and a polymer composition. Furthermore, polymer formulations may also be obtained by blending or melt-mixing at least one polymer or at least one polymer composition with additives, in particular non-polymeric additives. In all of these alternatives, polymer compounding is usually done to modify the properties of the polymeric starting materials in order to achieve desired characteristics.

Extrusion steps

The preparation of a melt in step (a) may comprise or consist of kneading a melt of a first polymer or polymer composition of at least one polymer and optional additives in an extruder. Steps (a) and (b) preferably constitute extrusion steps. Both steps may be carried out in a mixing device and/or extruder. The extruder may be a screw extruder. The extruder can be a single-screw or twin-screw extruder. The twin-screw extruder can be a co- or counter-rotating twin-screw extruder. A counter-rotating twin-screw extruder is, for instance, manufactured by Kobe and Japan Steel Works. A suitable example of an extruder is disclosed in EP-A-1600276.

The specific energy input (SEI) during step (a), is preferably within the range of 100 to 230 kWh/ton, wherein the weight refers to the total weight of the melt of the polymer or polymer composition and the optional additive(s). The screw speed of the extruder is preferably 140 rpm to 450 rpm, more preferably 170 rpm to 400 rpm and even more preferably 190 rpm to 380 rpm.

In step (b), the melt is passed through a die or several dies, such as in a die plate, in order to obtain the extruded melt. It is understood that a die plate is a thick metal disk having multiple dies (also called "holes"), wherein the holes are parallel to the screw axis of the extruder or mixing device. The form of the die (or hole) may determine the outer diameter of the extruded melt. However, the diameter and geometry of the die is not particularly limited. Furthermore, the extruded melt that is obtained after the melt has passed through the die or die plate can also be commonly referred to as "extrudate".

Steps (a) and (b) are preferably carried out at elevated temperature, for example at a temperature of at least 80°C, in particular of at least 100 °C, above the melting point or Vicat softening point of the at least one polymer or polymer composition. In a preferred embodiment, the temperature of the melt of the polymer or polymer composition may be within a range from 150 to 300 °C, more preferably within a range from 170 to 270 °C, even more preferably within a range from 180 to 250 °C.

Beforehand, i.e. prior to step (a), the polymer or polymer composition and the optional additives (hereinafter also "materials") may be fed into the mixing device and/or extruder in a solid form. If so, the materials may be heated to the desired temperature within a first zone of the mixing device and/or extruder. Alternatively, the materials may be fed into the mixing device and/or extruder in a molten state or in a combination, i.e. some materials in a molten state and others in a solid form.

Cooling

After the melt has been extruded through the die it automatically cools down. However, the extruded melt may be further cooled down in a cooling step. If so, the cooling step preferably includes decreasing the temperature below the solidification temperature of the extruded melt. The extruded melt may be cooled in air. Alternatively, the extruded melt may be passed through a water bath either before the cutting step (c) or during the cutting step (c). In the latter case, the cutting step is performed in an underwater pelletizer.

Cutting and collection steps

In step (c) the extruded melt is cut into pellets. Afterwards the pellets are collected in step (d). The cutting step (c) may also be referred to as a shaping step or a pelletizing step. Before or during the pelletizing step the extruded melt may need to be cooled down in a cooling step. This can either be done before pelletizing, for example by passing the extruded melt through cold ambient air or by passing the extruded melt through a water bath or it can be done during pelletizing, namely by means of an underwater pelletizer. The shape of the pellets is not particularly limited. However, pellets having a cylindrical form are well known.

FT-NIR measurement

Step (e) in the method for calibration according to the present invention relates to obtaining spectral information with an FT-NIR sensor. The FT-NIR sensor may include a detection region, an illumination source, and a detector. The sample, i.e. the extruded melt, may be illuminated with NIR light from at least one NIR illumination source in the detection region. The NIR light that is reflected by the sample is preferably received by the detector (reflection NIR spectroscopy). Alternatively, but less preferred, the spectral information can also be obtained in transmission by arranging illumination source and detector on opposite sides of the sample. Furthermore, in FT-NIR, *i.e.* the Fourier transform variant of NIR analysis, an interferometer is used to be able to obtain spectral information over a wide wavelength range in one instant, i.e. without wavelength scanning. Subsequent computer processing may turn the raw data collected by the FT-NIR sensor into spectral data for each wavelength so as to obtain a NIR spectrum. The obtained spectral information may relate to a wavelength range of from 800 to 2500 nm, preferably of from 900 to 1700 nm, in particular of from 950 to 1700 nm.

Depending on the position and operation of a sensor, measurement during a process can be termed "in-line", "on-line" or "off-line". With in-line measurement, measurements are taken directly in the process. In-line process analytics usually take place continuously, i.e. as long as the production process is running, automatic measurements can be made over and over again. During on-line measurement, measurements also usually take place continuously. However, on-line measurements are not made directly in the process, but rather in a built-in branch (or "sampling loop", or "bypass") into which samples of the substance to be measured, such as samples of a process fluid, are automatically fed. With off-line measurement, samples are manually taken from the process and assessed in a separate measuring device. Measurement may occur some time after processing, so direct process control is not possible.

In the present invention, the spectral information is obtained during compounding. More specifically, the FT-NIR spectra are measured in real-time directly on the extruded melt or on the pellets in steps b) to d)). Therefore, the spectral information is obtained "in-line" with the FT-NIR sensor. This provides an excellent means to allow real-time adjustment of processing parameters and to quickly differentiate between high- and low-quality compounds. The use of in-line FT-NIR detection reduces the necessity of performing time consuming quality control after the compounding process (off-line analysis), which reduces the resources required during testing and reduces waiting times during product development timelines. Overall, the use of in-line detection saves time and money compared to when off-line quality control processes are used.

Measuring the FT-NIR spectra on the extruded melt or the pellets in steps b) to d) allows to arrange the FT-NIR sensor to be located separate from or external to the compounding apparatus. For example, the FT-NIR sensor may be outside the extruder or mixing device. Furthermore, the FT-NIR sensor is preferably neither in contact with the extruded melt nor with the pellets. The only interaction of the FT-NIR sensor with the extruded melt or the pellets in steps b) to d) is through irradiation with light. This is opposed to an arrangement where an NIR probe is fitted into a bore hole to interact with the melt inside the extruder or mixing device. An outside or external arrangement of the FT-NIR sensor lessens the impact of fluctuating compounding conditions on the FT-NIR detection. Given that FT-NIR spectroscopy is highly dependent on parameters such as temperature, this allows consistent spectroscopic detection and calibration across variations in these parameters. In addition, the melt temperature and processing conditions of the compounding process can be altered without impacting the FT-NIR detection and calibration. Accordingly, this also enables the use of commercial FT-NIR sensors for over-the-belt type measurements, such as the DA7440 from Perkin Elmer, in the method according to the present invention.

Repetition and establishment of calibration curve

Steps f) requires the repetition of steps a) to e) for at least one further polymer or polymer composition having a known value of the at least one analytical feature. Step f) may comprise repeating steps a) to e) for at least two, preferably at least three, more preferably at least four further polymers or polymer compositions having a known value of the at least one analytical feature. For example, step f) may comprise repeating steps a) to e) for two to ten, such as three to seven further polymers or polymer compositions having a known value of the at least one analytical feature. It can be beneficial for the method for calibration when the at least one further polymer or polymer composition differs from the first polymer or polymer composition in only a single analytical feature. For example, if the analytical feature is the ethylene content, it is preferred to provide an ethylene copolymer as a further polymer that is identical to a first ethylene copolymer in all analytical features (such as content of non-polymeric additives) except for the ethylene content.

In step g) a calibration curve is established based on at least a part of the spectral information of the polymers or polymer compositions. Preferably, step g) comprises relating the known values of the at least one analytical feature to the spectral information of the polymers or polymer compositions from the extruded melt or the pellets, i.e. to the FT-NIR spectra of the polymers or polymer compositions.

Polymer or polymer composition

The polymer or polymer composition of at least one polymer may comprise a polyolefin homo- or copolymer, preferably a polyethylene or polypropylene homo- or copolymer.

The polymer or polymer composition may be a thermoplastic polymer or polymer composition. In one embodiment, the polymer may be a polyolefin homopolymer or a polyolefin copolymer. The polyolefin homopolymer or polyolefin copolymer may be selected from a virgin polyolefin homopolymer or polyolefin copolymer or a recycled polyolefin homopolymer or polyolefin copolymer or of a blend of those. Preferably, the polymer is a polyethylene homo- or copolymer or a polypropylene homo- or copolymer. More preferably, the polymer is a polypropylene homopolymer. Within the present invention, the term "copolymer" is not only used to cover polymers consisting of two different monomers but also higher order polymers consisting of three different monomers (terpolymers) or even more than three different monomers. The polymer or polymer composition may be a heterophasic copolymer. The heterophasic copolymer may be a heterophasic polypropylene copolymer.

A virgin polyolefin within the present invention denotes a material that is newly produced, in particular from monomeric units such as alpha olefins. The term "virgin" may, furthermore, apply to a material which has not yet completed a first use cycle and/or has not yet been recycled. In contrast to that, a recycled polyolefin is preferably recovered from waste plastic material derived from post-consumer and/or post-industrial waste. Post-consumer waste refers to objects having completed at least a first use cycle (or life cycle), i.e. having already served their first purpose; while industrial waste refers to manufacturing scrap, which does not normally reach a consumer.

The polymer composition preferably comprises more than 60 wt.-% of a polyolefin homopolymer, a polyolefin copolymer, mixtures or blends thereof, wherein the weight ratio refers to the total weight of the polymer composition and any optional additive(s). Further preferably, the polymer composition comprises more than 60 wt.-% of a polyethylene homo- or copolymer, a polypropylene homo- or copolymer, mixtures or blends thereof, wherein the weight ratio refers to the total weight of the polymer composition and any optional additive(s). In a particular embodiment, the polymer composition comprises more than 60 wt.-% of a propylene homopolymer.

The polymer composition preferably comprises more than 80 wt.-%, such as more than 90 wt.-%, in particular more than 95 wt.-%, of a polyolefin homopolymer, a polyolefin copolymer, mixtures or blends thereof, and less than 20 wt.-%, such as less than 10 wt.-%, in particular less than 5 wt.-%, of a non-polyolefin polymer, which can for example be considered a non-polyolefin polymeric contaminant, and wherein the weight ratio refers to the total weight of the polymer composition and any optional additive(s). The non-polyolefin polymer may be selected from the group consisting of elastomers, polystyrene homopolymers and copolymers, polyethylene terephthalate homopolymers or copolymers, polyamide homopolymers and copolymers, ethylene vinyl acetate copolymers, urethane polymers, mixtures and blends thereof. Preferably, the non-polyolefin polymer is selected from the group consisting of elastomers, polystyrene homopolymers and copolymers, polyethylene terephthalate homopolymers or copolymers, polyamide homopolymers and copolymers, mixtures and blends thereof.

The polymer or polymer composition may comprise at least one additive, preferably a non-polymeric additive. Purpose of the additive may be to enhance properties like color, flammability, conductivity or to modify rheology and other mechanical properties. The additive may already be present in the polymer or polymer composition before step a) of the method according to the invention. Alternatively, optional additives, *i.e.* at least one optional additive, may be melt-kneaded with a polymer that is free of additives or a polymer composition that is free of additives in the extruder in step a). The total content of the additive is preferably from 0.01 to 1 wt.-%, more preferably from 0.05 to 0.5 wt.-%, based on the total weight of the polymer or polymer composition.

Additives

Suitable additives are well known in the art and can, for example, be selected from the group consisting of antioxidants, anti-acids, antiblocking agents, UV protecting agents, nucleating agents and antistatic agents and combinations thereof, with antioxidants being the most preferred in the context of the present invention.

Examples of antioxidants which are commonly used in the art, are sterically hindered phenols (such as CAS No. 6683-19-8, also sold as Irganox 1010 FF™ by BASF, or Irganox 225™ by BASF), phosphorous based antioxidants (such as CAS No. 31570- 04-4, also sold as Hostanox PAR 24 (FF)™ by Clariant, or Irgafos 168 (FF)TM by BASF), sulphur based antioxidants (such as CAS No. 693- 36-7, sold as Irganox PS- 802 FL™ by BASF), nitrogen-based antioxidants (such as 4,4'- bis(1,1'-dimethyl- benzyl)diphenylamine), or antioxidant mixtures.

Anti-acids (so-called acid scavengers) are also commonly known in the art. Examples are calcium stearates, sodium stearates, zinc stearates, magnesium and zinc oxides, synthetic hydrotalcite (e.g. SHT, CAS-No. 11097-59-9), lactates and lactylates, as well as calcium stearate (CAS No. 1592-23-0) and zinc stearate (CAS No. 557-05-1).

Common antiblocking agents are natural silica such as diatomaceous earth (such as CAS No. 60676-86-0 (SuperfFloss™), CAS-No. 60676-86-0 (SuperFloss E™), or CAS-No. 60676-86-0 (Celite 499™)), synthetic silica (such as CAS-No. 7631-86-9, CAS-No. 7631-86-9, CAS-No. 7631-86-9, CAS-No. 7631-86-9, CAS-No. 7631-86-9, CAS-No. 7631-86-9, CAS-No. 112926-00-8, CAS-No. 7631-86-9, or CAS-No. 7631-86-9), silicates (such as aluminum silicate (Kaolin) CAS-no. 1318-74-7, sodium aluminum silicate CAS-No. 1344-00-9, calcined kaolin CAS-No. 92704-41-1, aluminum silicate CAS-No. 1327-36-2, or calcium silicate CAS-No. 1344-95-2), synthetic zeolites (such as sodium calcium aluminosilicate hydrate CAS-No. 1344-01-0, CAS-No. 1344-01-0, or sodium calcium aluminosilicate, hydrate CAS-No. 1344-01-0).

UV protecting agents are, for example, Bis-(2,2,6,6-tetramethyl-4-piperidyl)-sebacate (CAS-No. 52829-07-9, Tinuvin 770); 2-hydroxy-4-octyloxy-benzophenone (CAS-No. 1843-05-6, Chimassorb 81).

Nucleating agents like sodium benzoate (CAS No. 532-32-1); 1,3:2,4-bis(3,4-dimethylbenzylidene)sorbitol (CAS 135861-56-2, Millad 3988).

Suitable antistatic agents are, for example, glycerol esters (CAS No. 97593-29-8) or ethoxylated amines (CAS No. 71786-60-2 or 61791-31-9) or ethoxylated amides (CAS No. 204-393-1).

It is to be understood that further additives may also be included in the polymer or polymer composition and that the sum of all ingredients always adds up to 100 wt.-% in each of the embodiments described herein.

Analytical features

In the method according to the present invention, the at least one analytical feature may be selected from the group consisting of (1) the content of polymeric contaminants based on the total weight of the polymer or polymer composition, preferably the content of non-polyolefin polymeric contaminants, such as the content of elastomer, polystyrene, polyethylene terephthalate and/or polyamide contaminants, (2) the content of non-polymeric contaminants based on the total weight of the polymer or polymer composition, preferably the content of organic non-polymeric contaminants, (3) the C2 content, measured by solution state NMR as described below, and combinations thereof.

The content of polymeric contaminants (1) may also be referred to as the content of non-polyolefin polymer(s). The content of polymeric contaminants (1) may be from 0.01 wt.-% to 20 wt.-%, more preferably 0.5 wt.-% to 10 wt.-%, most preferably 0.1 wt.% to 5.0 wt.-%, based on the total weight of the polymer or polymer composition.

The content of non-polymeric contaminants (2) may also be referred to as the content of additives. The content of non-polymeric contaminants (2) may be from 0.01 wt.-% to 20 wt.-%, more preferably 0.5 wt.-% to 10 wt.-%, most preferably 0.1 wt.-% to 5.0 wt.-%, based on the total weight of the polymer or polymer composition.

The C2 content (3) refers to the ethylene content, indicated as weight percentage in the polymer or polymer composition. In case the polymer composition comprises more than 60 wt.-% of a propylene homopolymer, the C2 content (3) may be from 0.05 wt.-% to 40 wt.-%, more preferably 0.1 wt.-% to 20 wt.-%, most preferably 0.5 wt.-% to 18 wt.-%, based on the total weight of the polymer or polymer composition. The C2 content can be measured by solution state NMR as described below.

In another embodiment, the at least one analytical feature may also be the microstructure, determined from a combination of the C2 content and the C2 distribution, both measured by solution state NMR as described in the specification.

The microstructure of the polymer or polymer composition can relate to the covalent arrangement of different monomer units in the polymer chain, which may be classified as a random or block microstructure. The microstructure may be determined from a combination of the C2 content and the C2 distribution, both can be measured by established techniques including solution state NMR as described below.

Data Manipulation

The method for calibration using at least a part of the spectral information to establish a calibration curve, may further comprise at least one of the following steps (g1) preprocessing the spectral information, preferably by multiplicative scatter correction, standard normal variate transformation and/or by means of the Savitzky-Golay method, (g2) analyzing the spectral information, preferably by multivariate analysis, in particular by means of PLS and/or PCA multivariate analysis.

Both steps (g1) and (g2) may be considered data manipulation steps.

Preprocessing of the obtained spectral information (g1) can be used to optimize the subsequent analysis by removing unwanted variation that might impair the interpretation or predictive ability of the ensuing calibration curve, without excluding or altering chemically relevant variation. Preprocessing the spectral information is further advantageous because it can remove parasitic effects such as light straying caused by irregularities in the polymer or polymer composition.

Multiplicative scatter correction (MSC) is a widely used normalization technique. The transformation method can compensate for additive and/or multiplicative effects in spectral data, such as by removing the problems of baseline due to light scattering. In MSC, spectra may be corrected so they are as close as possible to a reference spectrum, generally the mean of the data set. This can be done by changing the scale and the offset of the spectra.

Standard normal variate (SNV) transformation is another a widely used normalization technique which may reduce multiplicative effects of scattering and particle size and may reduce differences in the global intensities of the signals. In SNV, each spectrum is centered and then scaled by dividing by its standard deviation.

With the Savitzky-Golay method or filter, data points may be smoothed to increase the precision of the data without distorting the signal tendency. A process known as convolution fits successive sub-sets of adjacent data points with a low-degree polynomial by the method of linear least squares. Preferably, the first and second derivative of the Savitzky-Golay method may be used when preprocessing the spectral information in step (g1).

Preprocessing may also include a step of discarding wavelength regions with irrelevant spectral information, which may be done by analyzing absorption spectra of extruded melts with different polymers or polymer compositions. In particular, those wavelength regions with the highest difference and the lowest signal noise should be selected to achieve a high predictive ability of the ensuing calibration curve.

The obtained spectral information of the polymer or polymer composition samples can then be analyzed in step (g2) before establishing the calibration curve. Analyzing the data in step (g2) can be helpful since the chemical information may be hidden twofold within the spectral information: Firstly, it may be hidden within the band positions. Band positions may give information about the presence of certain chemical compounds, such as non-polyolefin polymers in polymer compositions. Secondly, band intensities may carry quantitative information of these chemical compounds and may be a measure of the weight content.

In order to use the spectral information in a targeted way, it is necessary to find a relationship between the spectral information and the concentrations of certain chemical compounds. Whereas sometimes this may be straightforward and may be done by measuring the change of the intensity of a well resolved band that clearly belongs to the compound in question, it is not easily possible with the spectral information obtained with the FT-NIR sensor in the present invention.

Polymer compositions are a mixture of different chemical compounds and, therefore, tend to give spectra with overlapping bands so that intensities are not directly related to the content of a single compound. Therefore, chemical information in the method according to the present invention is preferably extracted from the spectral information by an analysis which is a multivariate analysis. The multivariate analysis is preferably a partial least squares analysis (PLS) and/or a principal component analysis (PCA).

Partial least squares analysis or regression (PLS) is a commonly used multivariate statistical technique. The regression method allows comparison between multiple response variables and multiple explanatory variables. In PLS, predictors are reduced to a smaller set of uncorrelated components and a least squares regression is performed on these components instead of the original data.

Principal component analysis (PCA) is another commonly used multivariate statistical technique. PCA is a linear dimensionality reduction technique used to simplify a large data set into a smaller set while still maintaining significant patterns and trends.

Cross-validation

The method for calibration may further comprise (h) a cross-validation step, wherein the accuracy of the established calibration curve is tested against a set of cross-validation polymers or polymer compositions.

This optional cross-validation step (h) may be put into the following context: The calibration curve is established on the basis of spectral information from at least two different polymers or polymer compositions with known values of at least one analytical feature. Preferably, the calibration curve is established on the basis of NIR spectral information from a first set of different polymers or polymer compositions with known values of at least one analytical feature. This first set of different polymers or polymer compositions may be termed a training set. To doublecheck the predictive ability and/or accuracy of the calibration curve, spectral information from a set of cross-validation polymers or polymer compositions with known values of at least one analytical feature (a second set of different polymers or polymer compositions) is collected, values of their analytical features are determined, i.e. predicted, and compared with their "true" values, i.e. their known values. It is understood that the cross-validation polymers or polymer compositions are preferably different from the polymers or polymer compositions in the training set in at least one analytical feature.

The predictive ability and/or accuracy of the calibration curve can be evaluated in terms of the root mean square error of cross validation (RMSEC) or the coefficient of determination ($R^2$) values.

The RMSEC value is calculated according to formula (A) below:

$$\text{RMSEC} = \sqrt{\frac{\sum_{i=1}^{n}(Y_{i_{measured}} - Y_{i_{predicted}})^2}{n}} \qquad (A)$$

where $Y_{measured}$ and $Y_{predicted}$ are the measured and predicted values, respectively. A low RMSEC value is an indicator for a high predictive ability and/or accuracy of the calibration curve.

The coefficient of determination ($R^2$) value is calculated according to formula (B) below:

$$R^2 = 1 - \frac{\sum_{i=1}^{n}(Y_{i_{prediceted}} - \overline{Y_{predicted}})^2}{\sum_{i=1}^{n}(Y_{i_{measured}} - \overline{Y_{measured}})^2} \qquad (B)$$

where $\overline{Y_{measured}}$ and $\overline{Y_{predicted}}$ are the measured and predicted average values, respectively. $R^2$ is preferably above 0.9 and is even more preferably above 0.95 for a calibration curve with a high predictive ability and/or accuracy.

The method for calibration may further comprise (i1) adding the spectral information of the set of cross-validation polymers or polymer compositions to the spectral information from which the calibration curve was obtained in order to create an extended data basis for calibration, and (i2) establishing an updated calibration curve based on the extended data basis after cross-validation step (h). The predictive ability and/or accuracy of the updated calibration curve is improved compared to the pervious calibration curve due to a larger data basis. In other words, the use of an extended data basis improves the predictive power of the calibration curve.

Method for in-line quality control

The present invention further relates to a method for in-line quality control in a compounding process comprising the following steps: (a') preparing a melt of a polymer or polymer composition of at least one polymer and optional additives in an extruder, (b') extruding the melt through a die of the extruder, (c') cutting the extruded melt into pellets, (d') collecting the pellets, (e') obtaining spectral information of the polymer or polymer composition from the extruded melt or the pellets in steps (b') to (d') with an FT-NIR sensor, and (f') determining the value of at least one analytical feature of the polymer or polymer composition based on a previously obtained calibration curve. Compared to the conventional off-line analysis, in-line quality control in compounding processes is cost-effective and contributes to a sustainable overall production concept. Deviations from the desired operation are recognized rapidly and countermeasures can be immediately taken.

The preferred features defined for steps (a) to (e) in the method for calibration are equally applicable to steps (a') to (e') in the method for in-line quality control, respectively.

Step (f) in the method for in-line quality control according to the present invention relates to the determination, *i.e.* prediction, of the (beforehand unknown) value of the at least one analytical feature of the polymer or polymer composition. This is achieved by comparison of the obtained FT-NIR spectral information with a previously obtained calibration curve that relates the spectral information to the value of the at least one analytical feature. The previously obtained calibration curve may be the calibration curve established in the method for calibration according to the present invention. Preferably, the previously obtained calibration curve has been obtained by use of the same apparatus for extrusion and pelletizing as is used for steps (a')-(d') above. In another preferred embodiment, when the method for in-line quality control determines the value of more than one analytical feature, it accordingly makes use of more than one previously obtained calibration curve.

Digital modelling tool

The method for in-line quality control may further comprise inputting the value of the at least one analytical feature of the polymer or polymer composition, as determined in step (f), into a digital modelling tool configured to predict properties of the polymer or polymer composition after extrusion.

Feeding a digital modelling tool with the value of the at least one analytical feature of the polymer or polymer composition, as determined in step (f), to predict properties of the polymer or polymer composition after extrusion is beneficial as it can save resources on subsequent investigations. The predicted properties may be used to make a decision on the usefulness of the polymer or polymer composition after extrusion. For example, if the polymer or polymer composition is a candidate composition which is suspected to satisfy the demands of a target application, the properties predicted by the digital modelling tool may bring clarity. The predicted properties may further be used to determine whether the respective polymer or polymer compositions are taken to the next research stage. Also, the use of the digital modelling tool may prove helpful for controlling a process in the development of application-tailored polymer composition, for example a compounding process.

Controller

The method for in-line quality control may further comprise inputting the value of the at least one analytical feature of the polymer or polymer composition, as determined in step (f), into a controller configured to adaptively control any of steps (a')-(d'), preferably at least step (a'), in particular based on the speed of the extrusion screws and/or the extrusion temperature.

Use of "in-line" FT-NIR detection allows for prompt control of the compounding process such as in this embodiment, where a controller is configured to adaptively control the steps of the compounding process. The controller can be configured to adaptively control the preparation of a melt of a polymer or polymer composition of at least one polymer and optional additives in an extruder. The controller can be configured to adaptively control the extrusion of the melt through a die of the extruder. The controller can be configured to adaptively control the cutting of the extruded melt into pellets. The controller can be configured to adaptively control the collection of the pellets. Preferably, the controller is configured to adaptively control the preparation of a melt of a polymer or polymer composition of at least one polymer and optional additives in an extruder. More preferably, the controller is configured to adaptively control the speed of screws in the extruder and/or the temperature in the extruder. The controller may be configured to adaptively control the amounts of the polymer, the polymer composition and the optional additives that are fed into the extruder. In one embodiment, the controller may be a PID-controller.

Apparatus

The present invention further relates to an apparatus for extrusion and pelletizing which comprises: (i) an extruder having a feed zone, a melting zone, a kneading zone and a discharge zone with a die; (ii) a pelletizer having a cutter configured to cut the extruded melt into pellets and a collection station for collecting the pellets; (iii) an in-line FT-NIR sensor installed outside of the extruder and outside of the pelletizer.

Extruder

Extruders are conveying devices that, according to the working principle of the Archimedean screw, evenly press solid to viscous masses out of a shaping opening under high pressure and high temperature. Usually, an extruder comprises a screw shaft, also called a screw. It is situated in the so-called barrel. The nominal diameter of the bore of the barrel may be equal to the outer diameter of the screw. At the downstream end of the barrel is a shaping opening, usually called die or die plate. On the other end of the barrel there may be a drive, in most cases an electric motor with a gear unit (extruder gear), which ensures rotation of the screw.

The extruder in the apparatus for extrusion and pelletizing according to the present invention has a feed zone where a polymer or polymer composition can be fed into the extruder. The feed zone can comprise a hopper. The hopper can be a gravity-fed hopper. The feed zone can interface with the melting zone via a feed throat.

The extruder further has a melting zone where a polymer or polymer composition can be melted to provide a polymer melt. The extruder may be a melt-extruder. The extruder is preferably a hot-melt extruder. The melting zone can melt polymers or polymer compositions through applied heat, friction and/or pressure. The melting zone can have a heating profile controlled by one or more controllers that gradually increases the temperature of the melting zone from the rear (where a polymer or polymer composition can enter) to the front. This allows for gradual heating of a polymer or polymer composition to avoid degradation.

The extruder has a discharge zone with a die where a polymer or polymer composition can be extruded. The die can form a polymer or polymer composition into a desired shape. The diameter and geometry of the die is not particularly limited and can govern the diameter and shape of the extruded melt.

The apparatus for extrusion and pelletizing according to the present invention further comprises a pelletizer which has a cutter that is configured to cut an extruded melt into pellets. The cutter can be a rotary, guillotine or fly knife cutter. The cutter can be other cutting machines known in the art. The cutter can comprise one or more blade or wire cutting sections. The pelletizer also has a collection station for collecting the resulting pellets.

The apparatus for extrusion and pelletizing according to the present invention comprises an in-line FT-NIR sensor. The FT-NIR sensor may include a detection region, an illumination source, a detector, and an interferometer. The sensor is installed outside of the extruder and outside of the pelletizer. The external location of the sensor lessens the impact of fluctuating compounding conditions on the FT-NIR detection. Given that FT-NIR spectroscopy is highly dependent on parameters such as temperature, this allows consistent spectroscopic detection and calibration across variations in these parameters. In addition, the melt temperature and processing conditions of the compounding process can be altered without impacting the FT-NIR detection and calibration.

The FT-NIR sensor may be an in-line FT-NIR sensor for over-the-belt type measurements. Such FT-NIR sensors are manufactured, for instance by PerkinElmer.

The apparatus for extrusion and pelletizing according to the present invention may further comprise a cooling bath between the extruder and the pelletizer. The cooling bath may comprise a water bath. The temperature of the cooling bath is not particularly limited.

**Brief Description of the Drawings**

Embodiments of the present invention are illustrated by way of example and are not limited by the figures of the accompanying drawings in which identical references represent similar elements.

**Fig. 1** shows a FT-NIR spectral overlay for homo polypropylene (PP) with 0.1 wt.-% to 5 wt.-% polyethylene terephthalate (PET).

**Fig. 2** shows a FT-NIR spectral overlay for homo polypropylene (PP) with 0.1 wt.-% to 5 wt.-% polystyrene (PS).

**Fig. 3** shows a FT-NIR spectral overlay for homo polypropylene (PP) with 0.1 wt.-% to 5 wt.-% polyamide (PA).

**Fig. 4** shows a FT-NIR spectral overlay for ethylene-propylene-copolymers with 0.5 wt.-% to 18 wt.-% C2 content (ethylene content).

**Fig. 5** is a correlation plot during cross-validation of a calibration curve for determining the content of polyethylene terephthalate (PET) in PP in a range from 0.1 wt.-% to 5 wt.-%.

**Fig. 6** is a correlation plot during cross-validation of a calibration curve used for determining the content of polystyrene (PS) in PP in a range from 0.1 wt.-% to 5 wt.-%.

**Fig. 7** is a correlation plot during cross-validation of a calibration curve for determining the content of polyamide (PA) in PP in a range from 0.1 wt.-% to 5 wt.-%.

**Fig. 8** is a correlation plot during cross-validation of a calibration curve for determining the ethylene content (C2 content) of an ethylene-propylene-copolymer in a range from 0.5 wt.-% to 18 wt.-%.

**Fig. 9** is a schematic of an embodiment of the apparatus for extrusion and pelletizing according to the present invention.

**Detailed Description of the Drawings and Examples**

The nature of the present invention will become more clearly apparent in view of the accompanying examples. The examples should, however, in no way limit the scope of the invention.

Materials

Polypropylene homopolymer (HC001A-B1, from Borealis AG), polyethylene terephthalate (recycled PET tray feedstock), polystyrene (POLYSTYROL 158 K Q, from BASF), polyamide (DOMAMID H36 LN, from DOMO Chemicals) polymers and different propylene-ethylene-copolymers were used as starting materials to compound the following samples. All polymers except PET were used as virgin grades. The propylene-ethylene-copolymers were produced at either pilot or full scale under a variety of conditions. Their ethylene contents were in a range from 0.4 wt.-% to 17.3 wt.-% in order to reflect the typical variety of copolymers encountered in the compounding process. The ethylene contents were determined by quantitative $^{13}$C solution-state NMR spectroscopy as outlined below.

Method for determination of C2-content in training set (propylene-ethylene-copolymers)

Quantitative $^{13}$C{$^1$H} NMR spectra were recorded in the solution-state using a Bruker Advance III 400 NMR spectrometer operating at 400.15 and 100.62 MHz for $^1$H and $^{13}$C respectively. All spectra were recorded using a $^{13}$C optimised 10 mm extended temperature probe head at 125 °C using nitrogen gas for all pneumatics. Approximately 200 mg of material was dissolved in 3 ml of 1,2-tetrachloroethane-$d_2$ (TCE-$d_2$) along with chromium-(III)-acetylacetonate (Cr(acac)$_3$) resulting in a 65 mM solution of relaxation agent in solvent {1}. To ensure a homogenous solution, after initial sample preparation in a heat block, the NMR tube was further heated in a rotatory oven for at least 1 hour. Upon insertion into the magnet the tube was spun at 10 Hz. This setup was chosen primarily for the high resolution and quantitatively needed for accurate ethylene content quantification. Standard single-pulse excitation was employed without NOE, using an optimised tip angle, 1 s recycle delay and a bi- level WALTZ16 decoupling scheme {2, 3}. A total of 6144 (6k) transients were acquired per spectra.

Quantitative $^{13}$C{$^1$H} NMR spectra were processed, integrated and relevant quantitative properties determined from the integrals using proprietary computer programs. All chemical shifts were indirectly referenced to the central methylene group of the ethylene block (EEE) at 30.00 ppm using the chemical shift of the solvent. This approach allowed comparable referencing even when this structural unit was not present. Characteristic signals corresponding to the incorporation of ethylene were observed {4}. The comonomer fraction was quantified using the method of Wang et. al. {5} through integration of multiple signals across the whole spectral region in the spectra. This method was chosen for its robust nature and ability to account for the presence of regiodefects when needed. Integral regions were slightly adjusted to increase applicability across the whole range of encountered comonomer contents. For systems where only isolated ethylene in PPEPP sequences was observed the method of Wang et al. was modified to reduce the influence of non-zero integrals of sites that are known to not be present. This approach reduced the overestimation of ethylene content for such systems and was achieved by reduction of the number of sites used to determine the absolute ethylene content to:

$$E = 0.5 \ (S\beta\beta + S\beta\gamma + S\beta\delta + 0.5(S\alpha\beta + S\alpha\gamma))$$

Through the use of this set of sites the corresponding integral equation becomes:

$$E = 0.5 \ (I_H + I_G + 0.5(I_C + I_D))$$

using the same notation used in the article of Wang et al. {5}. Equations used for absolute propylene content were not modified.

The mole percent comonomer incorporation was calculated from the mole fraction:

$$E\ [mol\%] = 100 * fE$$

The weight percent comonomer incorporation was calculated from the mole fraction:

$$E\ [wt\%] = 100 * (fE * 28.06\ ) / (\ (fE * 28.06) + ((l\text{-}fE) * 42.08)\ )$$

Bibliographic references:

1) Singh, G., Kothari, A., Gupta, V., Polymer Testing 28 5 (2009), 475.

2) Zhou, Z., Kuemmerle, R., Qiu, X., Redwine, D., Cong, R., Taha, A., Baugh, D. Winniford, B., J. Mag. Reson. 187 (2007) 225.

3) Busico, V., Carbonniere, P., Cipullo, R., Pellecchia, R., Severn, J., Talarico, G., Macromol. Rapid Commun. 2007, 28, 1128.

4) Cheng, H. N., Macromolecules 17 (1984), 19505) Wang, W-J., Zhu, S., Macromolecules 33 (2000), 1157. 7)

Instrumentation

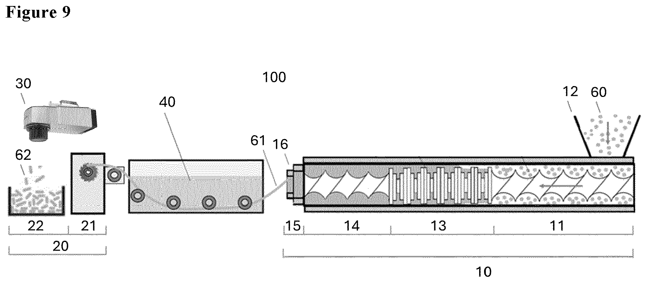

Samples were prepared with an apparatus having the setup as shown in fig. 9. The samples were compounded by extruding the starting materials in a ZSE 27 MAXX, LID 56 twin screw extruder supplied by Leistritz into a pellet water system. The feed rate throughput was set at 60 kg/h, with a screw speed of 600 rpm, a torque of 70% of the maximum torque and vacuum degassing at 300 mbar. The maximum temperature in the extruder, at which also melting was effected, was 230°C.

For in-line FT-NIR measurement a DA 7440 FT-NIR sensor from Perkin Elmer was used. The sensor was installed in-line after the cutter and at the collection station for collecting the pellets in order to collect NIR spectral information in real-time by detecting the reflected light. The FT-NIR sensor is based on core diode-array technology and is operative in the spectral range of 10,500 - 5800 cm$^{-1}$ (950-1700 nm) with a spectral resolution of 1.5 cm$^{-1}$.

Examples

A first set of training samples, namely polymer compositions comprising the following constituents, were compounded:

- PP/PET: Polypropylene homopolymer with 0 wt.-%, 0.1 wt.-%, 0.5 wt.-%, 1.0 wt.-%, 2.0 wt.-% and 5.0 wt.-% of polyethylene terephthalate (PET)
- PP/PS: Polypropylene homopolymer with 0 wt.-%, 0.1 wt.-%, 0.5 wt.-%, 1.0 wt.-%, 2.0 wt.-% and 5.0 wt.-% of polystyrene (PS)
- PP/PA: Polypropylene homopolymer with 0 wt.-%, 0.1 wt.-%, 0.5 wt.-%, 1.0 wt.-%, 2.0 wt.-% and 5.0 wt.-% of polyamide (PA)
- Propylene-ethylene-copolymer with 0.4 wt.-%, 0.8 wt.-%, 1.9 wt.-%, 2.0 wt.-%, 2.7 wt.-%, 4.4 wt.-%, 5 wt.-%, 5.4 wt.-%, 6.4 wt.-%, 7.4 wt.-%, 7.8 wt.-%, 9.3 wt.-%, 12.7 wt.-%, 13.9 wt.-%, 14.1 wt.-%, 17.3 wt.-% of C2 content

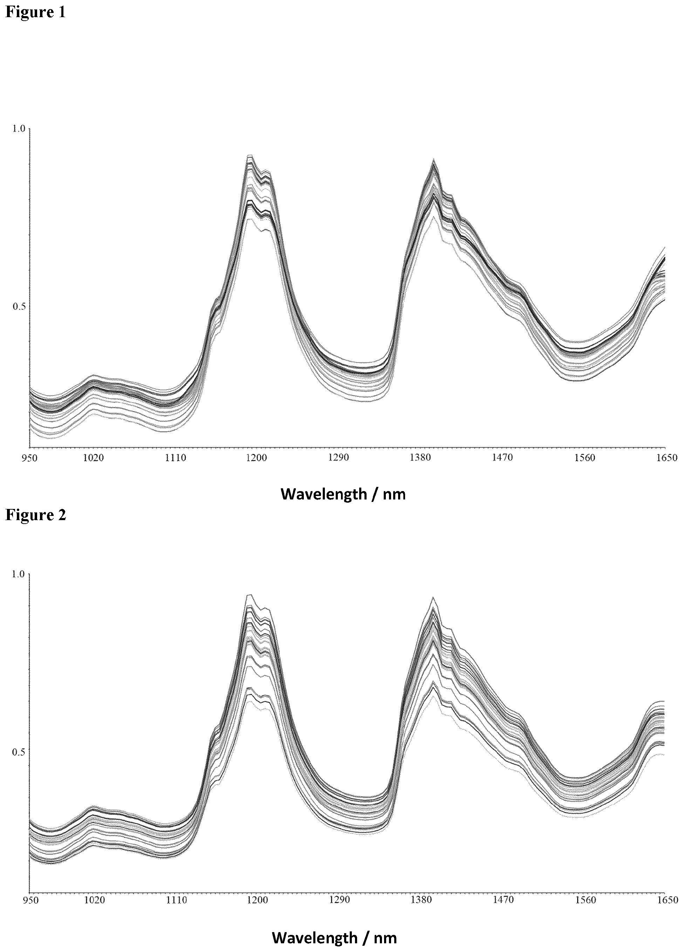

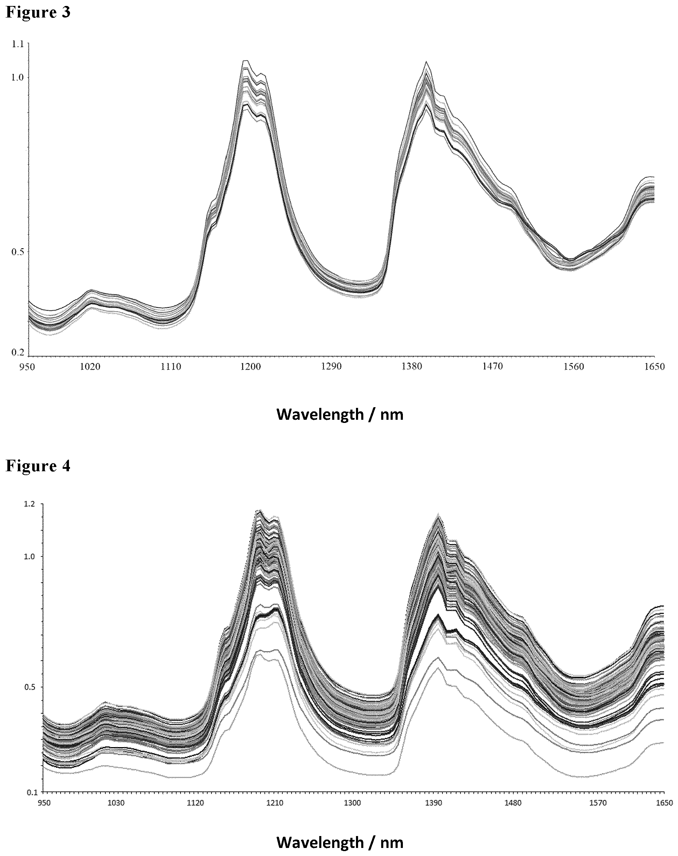

FT-NIR spectral information was measured during compounding and plotted together in the spectral overlay figures shown in **figures 1-4** where the wavelength in nanometers is shown on the x-axis and the non-normalized intensity in arbitrary units is shown on the y-axis.

Preprocessing and Analysis

The spectral information of the PP/PET, PP/PS and PP/PA samples was preprocessed using MSC, and by means of first or second derivative using the Savitzky-Golay method. The spectral information of the propylene-ethylene-copolymer samples was preprocessed using MSC, SNV transformation and by means of second derivative using the Savitzky-Golay method.

The spectral information was then analysed with multivariate analysis using PLS to develop calibration curves. PCA was further used to understand the number of influencing factors.

Cross-validation

Cross-validation of the calibration curves for PET, PS and PA content predictions was carried out with a second set of five validation samples. Three of the validation samples contained two of the following three non-polyolefin polymers: PET, PS and PA. Two validation samples contained even all three non-polyolefin polymers. The concentrations of PET, PS, PA in each of the five validation samples was known but varied randomly between 1 and 5 wt.-%. Likewise the calibration curve for the C2 content was validated with a second set of validation samples having propylene-ethylene copolymers with different but known ethylene contents.

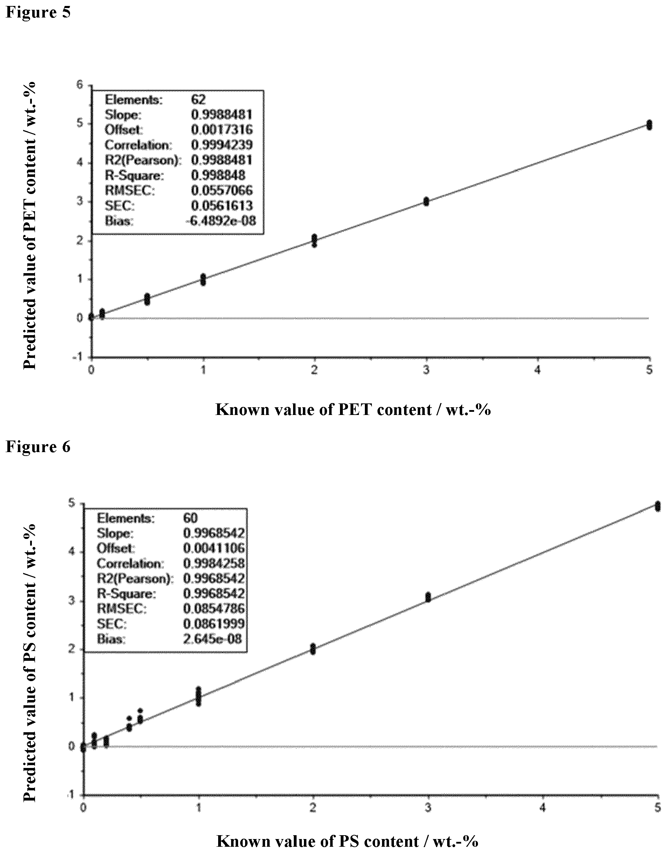

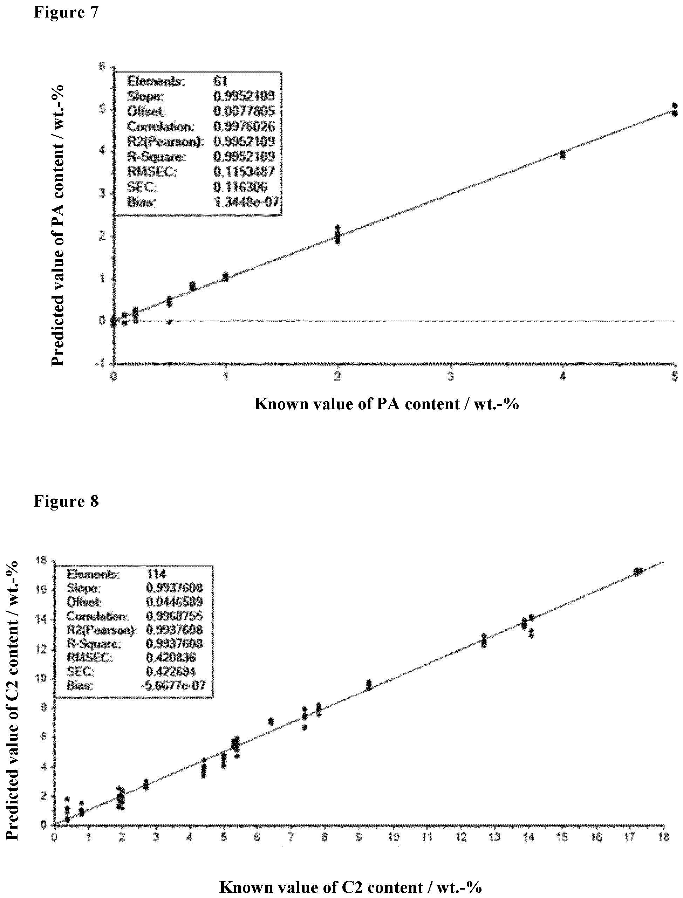

The results of the cross-validation are shown in the correlation plots in **figures 5-8.** As can be seen from the parameters listed in the left upper edge of the graph, in particular from the $R^2$ values (R-Square above 0.99) and from the RMSEC values (0.05 wt.-% to 0.11 wt.-% for PP/PET, PP/PS and PP/PA; 0.42 wt.-% for propylene ethylene copolymer), the predictive ability and accuracy of the calibration curves was excellent in all cases. Regarding the parameters it should be understood that whereas $R^2$ is dimensionless and varies between 0 and 1, RMSEC always has the same unit as the analyte. Therefore, RMSEC has the unit of wt.-% in the current case. Therefore, the value of RMSEC is not particularly limited. RMSEC of 0.42 wt.-%, for example, means that the resolution of the method is 0.42 wt%. Thus, with the calibration curve, one can successfully detect a deviation which is more than 0.42 wt.-% but values and deviations below 0.42 wt.-% will not be reliable. In other words, if the C2 content that is determined based on the calibration curve is 5.5 wt.-%, the true value can lie somewhere in between 5.1 to 5.9 wt.-%.

Generally, it is acknowledged that the accuracy will be improved by adding the spectral data of more polymer or polymer compositions to form an extended data basis and by then establishing an updated calibration curve.

An embodiment of the apparatus for extrusion and pelletizing according to the present invention will be described with reference to **figure 9.**

As shown in figure 9, an apparatus for extrusion and pelletizing 100 according to the present invention includes an extruder 10 having a feed zone 11 where the polymer or polymer composition 60 can be fed into the extruder 10. The feed zone 11 can comprise a hopper 12. The extruder 10 further comprises a melting zone 13, a kneading zone 14 and a discharge zone 15 with a die plate or die 16. The apparatus for extrusion and pelletizing 100 further includes a pelletizer 20 having a cutter 21 configured to cut the extruded melt 61 into pellets 62 and a collection station 22 for collecting the pellets 62. The apparatus for extrusion and pelletizing 100 further includes an in-line FT-NIR sensor 30 installed at the collection station 22 so as to obtain spectral information of the polymer or polymer composition during the collection of the pellets 62. Alternatively (not shown), the in-line FT-NIR sensor may be installed outside of the extruder 10, directly after the discharge zone 15, so as to obtain spectral information of the polymer or polymer composition from the just extruded melt 61. The apparatus for extrusion and pelletizing 100 may further comprise a cooling bath 40 between the extruder 10 and the pelletizer 20. In case the pelletizer 20 is an underwater pelletizer, the cooling bath 40 may be considered a part of the underwater pelletizer.

**Claims**

1. Method for calibration, comprising the following steps:

   a) preparing a melt of a polymer or polymer composition of at least one polymer and optional additives in an extruder,
   b) extruding the melt through a die of the extruder,
   c) cutting the extruded melt into pellets, and
   d) collecting the pellets,
   wherein the polymer or polymer composition has a known value of at least one analytical feature,
   the method being **characterized in that** it further comprises:

   e) obtaining spectral information of the polymer or polymer composition from the extruded melt or the pellets in steps b) to d) with an FT-NIR sensor,
   f) repeating steps a)-e) for at least one further polymer or polymer composition having a known value of the at least one analytical feature,
   g) using at least a part of the spectral information of the polymers or polymer compositions for establishing a calibration curve.

2. Method according to claim 1, wherein the polymer or polymer composition of at least one polymer comprises a polyolefin homo- or copolymer, preferably a polyethylene or polypropylene homo- or copolymer.

3. Method according to one of claims 1 or 2, wherein the at least one analytical feature is selected from the group consisting of:

    • the content of polymeric contaminants based on the total weight of the polymer or polymer composition, preferably the content of non-polyolefin polymeric contaminants, such as the content of elastomer, polystyrene, polyethylene terephthalate and/or polyamide contaminants,
    • the content of non-polymeric contaminants based on the total weight of the polymer or polymer composition, preferably the content of organic non-polymeric contaminants,
    • the C2 content, measured by solution state NMR as described in the specification,

and combinations thereof.

4. Method according to one of the preceding claims, wherein the at least one analytical feature is the microstructure, determined from a combination of the C2 content and the C2 distribution, both measured by solution state NMR as described in the specification.

5. Method according to one of the preceding claims, wherein using at least a part of the spectral information for establishing a calibration curve comprises at least one of the following steps:

    g1) preprocessing the spectral information, preferably by multiplicative scatter correction, standard normal variate transformation and/or by means of the Savitzky-Golay method,
    g2) analyzing the spectral information, preferably by multivariate analysis, in particular by means of PLS and/or PCA multivariate analysis.

6. Method according to one of the preceding claims, further comprising:
h) a cross-validation step, wherein the accuracy of the established calibration curve is tested against a set of cross-validation polymers or polymer compositions.

7. Method according to claim 6, further comprising:

    i1) adding the spectral information of the set of cross-validation polymers or polymer compositions to the spectral information from which the calibration curve was obtained in order to create an extended data basis for calibration, and
    i2) establishing an updated calibration curve based on the extended data basis after cross-validation step h).

8. Method for in-line quality control in a compounding process comprising the following steps:

    a') preparing a melt of a polymer or polymer composition of at least one polymer and optional additives in an extruder,
    b') extruding the melt through a die of the extruder,
    c') cutting the extruded melt into pellets,
    d') collecting the pellets,
    e') obtaining spectral information of the polymer or polymer composition from the extruded melt or the pellets in steps b') to d') with an FT-NIR sensor, and
    f) determining the value of at least one analytical feature of the polymer or polymer composition based on a previously obtained calibration curve.

9. Method according to claim 8, wherein the previously obtained calibration curve is the calibration curve established in the method according to one of claims 1-7.

10. Method according to one of claims 8 and 9, further comprising: inputting the value of the at least one analytical feature of the polymer or polymer composition, as determined in step f), into a digital modelling tool configured to predict properties of the polymer or polymer composition after extrusion.

11. Method according to one of claims 8-10, further comprising: inputting the value of the at least one analytical feature of the polymer or polymer composition, as determined in step f'), into a controller configured to adaptively control any of steps a')-d'), preferably at least step a'), in particular based on the speed of the extrusion screws and/or the extrusion temperature.

12. Apparatus for extrusion and pelletizing under in-line quality control, the apparatus comprising:

    (i) an extruder having a feed zone, a melting zone, a kneading zone and a discharge zone with a die;
    (ii) a pelletizer having a cutter configured to cut the extruded melt into pellets and a collection station for collecting the pellets;
    (iii) an in-line FT-NIR sensor installed outside of the extruder and outside of the pelletizer.

13. Apparatus according to claim 12, wherein the FT-NIR sensor is an in-line FT-NIR sensor for over-the-belt type measurements.

14. Apparatus according to one of claims 12 and 13 further comprising a cooling bath between the extruder and the pelletizer.

**Figure 1**

Wavelength / nm

**Figure 2**

Wavelength / nm

Figure 3

Wavelength / nm

Figure 4

Wavelength / nm

Figure 5

Known value of PET content / wt.-%

Figure 6

Known value of PS content / wt.-%

Figure 7

Figure 8

**Figure 9**

Europäisches
Patentamt

European
Patent Office

Office européen
des brevets

## EUROPEAN SEARCH REPORT

**Application Number**

EP 24 17 5513

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | FISCHER D ET AL: "In-line process monitoring on polymer melts by NIR-spectroscopy", FRESENIUS J ANAL CHEM, vol. 359, 1997, pages 75-77, XP093209817, * the whole document * | 1-14 | INV. G01N21/3563 G01N21/3577 G01N21/359 ADD. G01N21/84 G01N21/35 |
| Y | TAKEFUMI NAGATA ET AL: "In-line monitoring of polyethylene density using near infrared (NIR) spectroscopy", POLYMER ENGINEERING & SCIENCE, vol. 40, no. 5, May 2000 (2000-05), pages 1107-1113, XP055056121, ISSN: 0032-3888, DOI: 10.1002/pen.11238 * page 1106 * * page 1110 * * figure 1 * | 1-14 | |
| Y | HALL JEFFREY W. ET AL: "Analysis of Polymer Pellets Obtained from Two Extruders Using near Infrared Spectroscopy", JOURNAL OF NEAR INFRARED SPECTROSCOPY, vol. 1, no. 1, 1993, pages 55-62, XP093210328, GB ISSN: 0967-0335, DOI: 10.1255/jnirs.6 * pages 55, 61 * | 1-14 | TECHNICAL FIELDS SEARCHED (IPC) G01N |
| Y | US 5 468 586 A (PROPER JAMES M [US] ET AL) 21 November 1995 (1995-11-21) * column 7, lines 50-57 * * figure 1 * | 13 | |

-/--

The present search report has been drawn up for all claims

| Place of search | Date of completion of the search | Examiner |
|---|---|---|
| Munich | 2 October 2024 | Hoogen, Ricarda |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**page 1 of 2**

Europäisches
Patentamt

European
Patent Office

Office européen
des brevets

**EUROPEAN SEARCH REPORT**

Application Number

EP 24 17 5513

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MCPETERS H. LEE: "On-line analysis of polymer melt processes", ANALYTICA CHIMICA ACTA, ELSEVIER, AMSTERDAM, NL, vol. 238, 1990, pages 83-93, XP026725841, ISSN: 0003-2670, DOI: 10.1016/S0003-2670(00)80526-7 [retrieved on 1990-01-01] * figure 1 * | 14 | |
| A | DESA S ET AL: "In-line monitoring of particulate contaminants and composition in polymer extrusion", POLYMERIC MATERIALS SCIENCE AND ENGINEERING. PMSE PREPRINTS, AMERICAN CHEMICAL SOCIETY, US, vol. 72, 1995, pages 21-22, XP009088859, ISSN: 0743-0515 * the whole document * | 1-14 | |

TECHNICAL FIELDS
SEARCHED          (IPC)

The present search report has been drawn up for all claims

| Place of search | Date of completion of the search | Examiner |
|---|---|---|
| Munich | 2 October 2024 | Hoogen, Ricarda |

EPO FORM 1503 03.82 (P04C01)

## ANNEX TO THE EUROPEAN SEARCH REPORT
## ON EUROPEAN PATENT APPLICATION NO.

EP 24 17 5513

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2024

| Patent document cited in search report | Publication date | Patent family member(s) | Publication date |
|---|---|---|---|
| US 5468586 A | 21-11-1995 | NONE | |

For more details about this annex : see Official Journal of the European Patent Office, No. 12/82

## REFERENCES CITED IN THE DESCRIPTION

### Patent documents cited in the description

- WO 2015195479 A1
- ES 2346494 A1
- CN 201067517 Y
- EP 1600276 A

### Non-patent literature cited in the description

- **WITSCHNIGG, ANDREAS et al.** In-line characterization of polypropylene nanocomposites using FT-NIR.. *Journal of applied polymer science*, 2010, vol. 117 (5), 3047-3053
- **BARBAS, J. M.** ; **A. V. MACHADO** ; **J. A. COVAS.** In-line near-infrared spectroscopy for the characterization of dispersion in polymer-clay nanocomposites. *Polymer testing*, 2012, vol. 31 (4), 527-536
- **SAERENS, LIEN et al.** In-line NIR spectroscopy for the understanding of polymer-drug interaction during pharmaceutical hot-melt extrusion. *European Journal of Pharmaceutics and Biopharmaceutics*, 2012, vol. 81 (1), 230-237
- **FISCHER, D. et al.** In-line process monitoring on polymer melts by NIR-spectroscopy.. *Fresenius' journal of analytical chemistry*, 1997, vol. 359, 74-77
- **FISCHER, DIETER et al.** Process monitoring of polymers by in-line ATR-IR, NIR and Raman spectroscopy and ultrasonic measurements. *Comptes Rendus Chimie*, 2006, vol. 9 (11-12), 1419-1424
- *CHEMICAL ABSTRACTS*, 6683-19-8
- *CHEMICAL ABSTRACTS*, 31570- 04-4
- *CHEMICAL ABSTRACTS*, 693- 36-7
- *CHEMICAL ABSTRACTS*, 11097-59-9
- *CHEMICAL ABSTRACTS*, 1592-23-0
- *CHEMICAL ABSTRACTS*, 557-05-1
- *CHEMICAL ABSTRACTS*, 60676-86-0
- *CHEMICAL ABSTRACTS*, 7631-86-9
- *CHEMICAL ABSTRACTS*, 112926-00-8
- *CHEMICAL ABSTRACTS*, 1318-74-7
- *CHEMICAL ABSTRACTS*, 1344-00-9
- *CHEMICAL ABSTRACTS*, 92704-41-1
- *CHEMICAL ABSTRACTS*, 1327-36-2
- *CHEMICAL ABSTRACTS*, 1344-95-2
- *CHEMICAL ABSTRACTS*, 1344-01-0
- *CHEMICAL ABSTRACTS*, 52829-07-9
- *CHEMICAL ABSTRACTS*, 1843-05-6
- *CHEMICAL ABSTRACTS*, 532-32-1
- *CHEMICAL ABSTRACTS*, 135861-56-2
- *CHEMICAL ABSTRACTS*, 97593-29-8
- *CHEMICAL ABSTRACTS*, 71786-60-2
- *CHEMICAL ABSTRACTS*, 61791-31-9
- *CHEMICAL ABSTRACTS*, 204-393-1
- **SINGH, G.** ; **KOTHARI, A.** ; **GUPTA, V.** *Polymer Testing*, 2009, vol. 28 (5), 475
- **ZHOU, Z.** ; **KUEMMERLE, R.** ; **QIU, X** ; **REDWINE, D.** ; **CONG, R.** ; **TAHA, A.** ; **BAUGH, D.** ; **WINNIFORD, B.** *J. Mag. Reson.*, 2007, vol. 187, 225
- **BUSICO, V.** ; **CARBONNIERE, P.** ; **CIPULLO, R.** ; **PELLECCHIA, R.** ; **SEVERN, J.** ; **TALARICO, G.** *Macromol. Rapid Commun.*, 2007, vol. 28, 1128
- **CHENG, H. N.** *Macromolecules*, 1984, vol. 17, 19505
- **WANG, W-J.** ; **ZHU, S.** *Macromolecules*, 2000, vol. 33